(19) Europäisches Patentamt

European Patent Office

Office européen des brevets

(11) **EP 0 505 918 B1**

## (12) EUROPÄISCHE PATENTSCHRIFT

(45) Veröffentlichungstag und Bekanntmachung des Hinweises auf die Patenterteilung:
**04.12.1996 Patentblatt 1996/49**

(51) Int. Cl.$^6$: **A61B 5/029**

(21) Anmeldenummer: **92104746.0**

(22) Anmeldetag: **19.03.1992**

(54) **Vorrichtung und Verfahren zur Ermittlung des Herzzeitvolumens**

Apparatus and method for determining heart minute volumes

Dispositif et procédé pour déterminer les volumes éjectés par minute de coeur

(84) Benannte Vertragsstaaten:
**AT BE CH DE FR GB LI**

(30) Priorität: **25.03.1991 DE 4109720**

(43) Veröffentlichungstag der Anmeldung:
**30.09.1992 Patentblatt 1992/40**

(73) Patentinhaber: **Hoeft, Andreas, Dr. med.**
**53127 Bonn (DE)**

(72) Erfinder: **Hoeft, Andreas, Dr. med.**
**53127 Bonn (DE)**

(74) Vertreter: **Patentanwälte Thömen & Körner**
**Zeppelinstrasse 5**
**30175 Hannover (DE)**

(56) Entgegenhaltungen:
**DE-B- 1 498 534          DE-B- 2 622 726**

- **PROCEEDINGS OF THE SEVENTH ANNUAL CONFERENCE OF THE IEEE/ENGINEERING IN MEDICINE AND BIOLOGY SOCIETY Bd. 1, 27. September 1985, CHICAGO USA Seiten 490 - 494; N.E.FEARNOT ET AL: 'calculation of catheter-based saline dilution cardiac output'**

**Beschreibung**

Die Erfindung betrifft eine Vorrichtung zur Ermittlung des Herzzeitvolumens nach dem Oberbegriff des Anspruchs 1.

Das Herzzeitvolumen stellt einen der wichtigsten Einzelparameter zur globalen Beurteilung der Herzorganfunktion dar. Die Ermittlung dieser Größe kann sowohl unter wissenschaftlichen als auch unter klinischen Aspekten wertvolle Entscheidungskriterien liefern. Wichtige Anwendungsgebiete umfassen insbesondere die Überwachung und kardiovaskuläre Diagnostik kritisch Kranker, die Überwachung von Patienten während herzchirurgischer Operationen und anderer ausgedehnter operativer Eingriffe, die Verlaufskontrolle und Differentialindikationen beim Einsatz potenter hämodynamisch wirksamer Pharmaka sowie den Leistungssport. Darüberhinaus stellt das Herzzeitvolumen die Grundlage für die Ableitung einer Anzahl weiterer diagnostisch wichtiger Herz-Kreislauf-Parameter dar, wie z.B. der periphere Widerstand oder Herzarbeitsindizes.

Im Bereich des Leistungssports sind invasive Verfahren nicht geeignet, das Herzzeitvolumen unter Praxisbedingungen zu messen. Hier kommt es nämlich darauf an, die Bewegungsfreiheit und die Leistungsentfaltung des Sportlers so wenig wie möglich zu beeinträchtigen, damit realistische Werte gewonnen werden können, die wiederum zur Verbesserung und Überprüfung der Wirksamkeit der Trainingsmethoden herangezogen werden können.

Bei einem bekannten Verfahren zur Ermittlung des Herzzeitvolumens wird ein Indikator, z.B. eine gekühlte Lösung als Äquivalent für eine bestimmte Kältemenge oder ein Farbstoff über einen Katheter in den rechten Vorhof des Herzens injiziert und die resultierende Indikatorverdünnungskurve an einer oder mehreren Stellen der Arteria pulmonalis oder der Aorta gemessen. Die Berechnung des Herzzeitvolumens aus den gemessenen Indikatorverdünnungskurven basiert auf dem Prinzip der Massenerhaltung gemäß der nach Stewart Hamilton angegebenen Formel.

Die Anwendung dieses bekannten Verfahrens beinhaltet Belastungen und Risiken für den Patienten, da eine arterielle und/oder pulmonalarterielle Katheterisierung erfolgen muß. Auf Grund der hohen Invasivität dieses Verfahrens ist eine nicht zu vernachlässigende Morbidität gegeben.

Über die Anwendung des Verfahrens wird daher in Abwägung zwischen dem Informationsgehalt der abgeleiteten Daten und der jeweiligen Belastung und Risiken für den Patienten entschieden. Dadurch wird bei vielen Indikationen, bei denen die Ermittlung des Herzzeitvolumens an sich hilfreich und wünschenswert wären, auf die Messung dieses Wertes zugunsten einer Risikominderung verzichtet.

Aus der DE-PS 26 22 726 ist bereits eine Vorrichtung zur Bestimmung des Herzzeitvolumens auf der Basis der Farbstoffverdünnungstechnik bekannt, bei der die Konzentration des oxidierten Hämoglobins, des reduzierten Hämoglobins und eines Indikatorfarbstoffes bestimmt werden sollen. Die Bestimmung erfolgt über die Absorptionseigenschaften der Farbstoffe gegenüber Licht bestimmter Wellenlängen nach dem Gesetz von Lambert-Beer.

Voraussetzung für die Anwendbarkeit des Lambert-Beer'schen Gesetzes ist, daß eine ideale Farbstofflösung vorliegt, bei der sich die Photonen nicht beeinflussen, in der keinerlei Streuung des Lichtes auftritt und in der nur kleine Farbstoffkonzentrationen vorhanden sind. Diese Bedingungen sind in der Praxis nicht erfüllt, insbesondere liegen bei Transmissionsmessungen an biologischen Geweben mit offensichtlich starker Lichtstreuung diese Voraussetzungen nicht vor, so daß mit erheblichen Fehlern zu rechnen ist.

Aus der DE-B-14 98 534, aus der die Merkmale der Oberbegriffe der Ansprüche 1 und 8 hervorgehen, ist ferner ein Verfahren und eine Vorrichtung zur Bestimmung der Farbstoffverdünnung im Blut bekannt. Dabei sollen Schwankungen des Sauerstoffsättigungsgrades, die sich auf Lichtabsorptionsmessungen oder Lichtextinktionsmessungen auswirken, kompensiert werden. Gleichzeitig lassen sich auch weitere Störeinflüsse von zeitlichen Schwankungen kompensieren. Dieses Verfahren und die Vorrichtung kann bei der Bestimmung des Herzzeitvolumens verwendet werden.

Der Erfindung liegt die Aufgabe zugrunde, eine Vorrichtung zur Ermittlung des Herzzeitvolumens dahingehend zu verbessern, daß die Belastung und Risiken für den Patienten wesentlich verringert werden und dennoch genaue Meßergebnisse erzielt werden können.

Diese Aufgabe wird bei einer Vorrichtung nach dem Oberbegriff des Anspruchs 1 durch die im Kennzeichen angegebenen Merkmale gelöst.

Die eigentliche Messung mit der erfindungsgemäßen Vorrichtung ist nicht-invasiv, denn die Ermittlung der Farbstoffkonzentrationszeitkurve wird ausschließlich durch Licht-Transmissions- oder Reflexionsmessung vorgenommen, wobei sowohl das Licht von außen in den Meßort eingestrahlt wird als auch die Lichtintensität außen gemessen wird. Lediglich das Injizieren des Indikatorfarbstoffes in den Blutkreislauf erfordert noch einen Eingriff. In vielen Fällen ist aber bei Patienten, die sich operativer Eingriffe unterziehen, routinemäßig ein Katheter zur Injektion von Medikamenten angelegt, so daß dieser ohne zusätzliche Belastung für den Patienten mitbenutzt werden kann.

Die mit der erfindungsgemäßen Vorrichtung vorgenommene Messung ohne Eingriffe in den Körper beinhaltet das Problem, daß die für die Berechnung benötigte Kenntnis der Farbstoffkonzentration nicht unmittelbar über eine Licht-Transmissions- oder Reflexionsmessung möglich ist. Das optische Verhalten von jeglichen biologischen Geweben wird wesentlich durch Streuung des Lichtes determiniert. Das Lambert-Beer'sche Gesetz ist daher nicht anwendbar. Es liegen optische Verhältnisse vor, die zutreffender durch die Zwei-Fluß Theorie nach Kubelka und Munk beschrieben werden. Hieraus folgt, daß Farbstoffkonzentrationen in optisch streuenden Medien nicht in absoluter Konzentration gemessen werden können, daß jedoch bei Farbstoffgemischen die Verhältnisse der Farbstoffe zueinander ermittelt werden können. Dies bedeutet für das vorliegende Problem der nicht invasiven Lichttransmissions- bzw. Reflexionsmessung, daß der Meßwert zwar der Konzentrationszeitkurve des Indikatorfarbstoffes folgt, daß jedoch dieser Verlauf nur eine qualitative und keine quantitative Aussage erlaubt.

Die Erfindung beschreibt nun einen Weg, die Konzentration mittelbar zu erfassen. Dies geschieht über die Einbeziehung eines Referenzfarbstoffes, vorzugsweise des roten Blutfarbstoffes, da dieser bereits physiologischerweise im Blut vorhanden ist. Alternativ könnte jedoch auch ein anderer künstlich hinzugesetzter Farbstoff verwendet werden.

Der Referenzfarbstoff muß sich vollständig im Blutkreislauf verteilt haben und entweder eine konstante Konzentration oder aber einen Konzentrationsverlauf besitzen, dessen zeitliche Funktion bekannt ist. Im Falle des roten Blutfarbstoffes kann im Rahmen einer vorausgehenden Blutuntersuchung die Konzentration in vitro bestimmt werden.

Die Meßvorrichtung kann selektiv auf wenigstens zwei Wellenlängen abgestimmt sein, bei denen der Indikatorfarbstoff und der Referenzfarbstoff unterschiedliches Absorptionsverhalten aufweisen.

Weiterhin können die auszuwertenden Wellenlängen des eingestrahlten Lichtes so gewählt sein, daß eine der Wellenlängen im Absorptionsbereich des injizierten Indikatorfarbstoffes liegt, und eine andere Wellenlänge in einem Bereich liegt, in dem der Referenzfarbstoff und der Indikatorfarbstoff unterschiedliches Absorptionsverhalten aufweisen.

Die Auswahl der Wellenlängen entsprechend diesem Auswahlkriterium führt dazu, daß besonders deutliche Unterschiede des Absorptionsverhaltens zwischen dem Indikatorfarbstoff und dem Referenzfarbstoff auftreten, so daß sich die Meßwerte deutlich über einen nicht vermeidbaren Störpegel herausheben und außerdem nicht mit einer zu hohen Menge des Indikatorfarbstoffes gearbeitet werden muß.

Alternativ ist vorgesehen, daß bei Verwendung mehrerer Indikatorfarbstoffe und Referenzfarbstoffe Licht mit mehreren Wellenlängen oder mit einem kontinuierlichen Spektrum eingestrahlt wird und daß aus den Funktionen der gemessenen zeitlichen Schwankungen der Lichtintensitäten durch den Rechner mehrere Meßwertgrößen errechnet werden und eine Fehlerkorrektur und/oder Mittelwertbildung erfolgen kann.

Die Mindestvoraussetzung für die Auswertung unterschiedlicher Farbstoffe bildet die Verwendung von Licht zur Einstrahlung in den Meßort, das mindestens zwei Wellenlängen umfaßt. Es kann jedoch auch Licht verwendet werden, bei dem mehrere Wellenlängen oder ein ganzes Spektrum ausgewertet wird. Die unterschiedlichen Lichtintensitäten, die infolge der Transmissions- oder Reflexionsmessung erfaßt werden, stellen ein Maß für die relativen Konzentrationen der Farbstoffe dar, so daß über die bekannte Konzentration eines der Farbstoffe, also des Referenzfarbstoffes, auch die Konzentration des anderen, also des Indikatorfarbstoffes bestimmt werden kann.

Die unterschiedlichen Wellenlängen können so ausgewählt werden, daß vom Lichtsender her bereits eine Selektion erfolgt und nur Licht bevorzugter Wellenlängen ausgestrahlt wird. Es ist dann möglich, auf der Empfängerseite die Signale breitbandig auszuwerten. Andererseits besteht auch die Möglichkeit, den Lichtsender ein breites Lichtspektrum aussenden zu lassen und empfängerseitig eine Selektion durchzuführen. Darüberhinaus können sowohl auf der Sender- als auch auf der Empfängerseite Selektionen der gewünschten Wellenlängen erfolgen.

Bei dieser Vorgehensweise ermöglicht die erweiterte Auswertung mehrerer Wellenlängen oder spektrale Auswertung eine ständige Überprüfung der Meßergebnisse auf Plausibilität und eventuell auch eine Korrektur oder Mittelwertbildung in dem Falle, daß z.B eine Störung durch Artefakte eintritt. Des weiteren wären hierbei mehrere Farbstoffe als Indikator verwendbar, die u.U. unterschiedliche spezifische Eigenschaften aufweisen.

Bei praktischer Anwendung kann mit rotem Blutfarbstoff als Referenzfarbstoff und Indocyaningrün als Indikatorfarbstoff gearbeitet werden. Die Wellenlängen des eingestrahlten Lichtes werden so gewählt, daß einerseits im maximalen Absorptionsbereich des Indocyaningrüns bei 800 nm und andererseits im nichtabsorbierenden Infrarotwellenlängenbereich des Indocyaningrüns oberhalb von 900 nm gemessen wird.

Die Auswahl dieser Wellenlängen konnte bei bisherigen Versuchen optimale Ergebnisse hervorbringen, die auch durch Kontrollmessungen mit herkömmlichen Verfahren bestätigt wurden

Das Absorptionsverhalten der Farbstoffe wird auf der Basis zeitlicher Schwankungen der Lichtintensitäten auf Grund der pulsatilen Blutströmung in arteriellen und kapillären Gefäßabschnitten ermittelt. Dadurch ist eine Differenzierung gegenüber Absorptionseigenschaften des Gewebes und der nicht-pulsatilen, venösen Blutanteile ermöglicht.

Es gelingt so, den Basisanteil der Absorption zu eliminieren und den Vergleich lediglich auf den pulsatilen Anteil zu beschränken. Die relativen Unterschiede zwischen den auszuwertenden Lichtintensitäten auf Grund der Absorption durch den Indikatorfarbstoff einerseits und den Referenzfarbstoff andererseits werden so deutlicher, was der Meßgenauigkeit zugute kommt.

Während bei der eingangs erwähnten bekannten Vorrichtung die pulsatilen Anteile als störend für die Messung betrachtet werden, nutzt die erfindungsgemäße Vorrichtung gerade die pulsatilen Anteile für die Messung aus und erreicht so bei geringem Schaltungs- und Rechenaufwand eine hohe Genauigkeit.

Nunmehr ist es möglich, in Kenntnis der Konzentration des Referenzfarbstoffes durch vergleichende Messung auch die Konzentration des Indikatorfarbstoffes zu bestimmen. Unter Einbeziehung der in den Blutkreislauf injizierten Menge sind somit alle Größen bekannt, um nach der Formel von Stewart Hamilton das Herzzeitvolumen zu bestimmen.

Vorzugsweise ist die Meßwertgröße der Quotient aus den Funktionen der gemessenen zeitlichen Schwankungen der Lichtintensitäten und die Farbstoffkonzentrationszeitkurve stellt das Produkt aus der Meßwertgröße und der bekannten Konzentration des Referenzfarbstoffes dar.

Die Meßwertgröße und die Farbstoffkonzentrationszeitkurve können also mit wenig Rechenaufwand aus den Lichtintensitätsschwankungen kontinuierlich ermittelt werden, so daß der gesuchte Wert des Herzzeitvolumens unmittelbar nach Abschluß der Messung zur Verfügung steht.

Zur Verbesserung der Meßgenauigkeit können Einflüssen des verzögerten Indikatorfarbstoffeintritts in den jeweiligen Meßort, z.B. bei schlechter Durchblutung des jeweiligen Meßortes, durch durchblutungsverbessernde Maßnahmen entgegengewirkt werden.

Dadurch lassen sich z.B. die Auswirkungen einer Rezirkulation verringern, die dazu führen können, daß die Indikatorverdünnungskurve an seiner abfallenden Flanke verschmiert wird. Solch eine Erscheinung wird u.a. durch indikatorfarbstoffhaltige Blutanteile hervorgerufen, die nach Durchlaufen eines kürzeren Kreislaufes erneut zum Meßort gelangen.

Eine andere Alternative sieht vor, daß derartige Einflüsse durch erweiterte mathematische Auswertungsverfahren der Farbstoffkonzentrationszeitkurve des Indikatorfarbstoffes unter Anwendung eines längeren Zeitabschnittes kompensiert werden. Dabei umfaßt dieser Zeitabschnitt den Konzentrationsanstieg am Meßort, die Verteilungsphase des Farbstoffes im Blutkreislauf, die Rezirkulation und den Beginn der Farbstoffelimination. Die zusätzliche Information, die durch die längere Registrierung über den Rezirkulationsprozeß und über die Farbstoffelimination gewonnen werden kann, kann vorteilhaft zur Berechnung des Herzzeitvolumens ausgenutzt werden. Hierdurch ist es möglich, auch bei protrahierterem Konzentrationszeitverlauf der Indikatorverdünnungskurve die erste, für die Berechnung des Herzzeitvolumens entscheidende Passage des Indikators am Meßort von rezirkulierenden Indikatoranteilen zu trennen.

Dies hat den Vorteil, daß die Messung nicht von weiteren Behandlungsmaßnahmen begleitet werden muß, also z.B. die Erwärmung von Körperteilen oder die Verabreichung von durchblutungsfördernden Medikamenten unterbleiben kann. Vielmehr läßt sich die Auswertung rechnergestützt durchführen, wobei der für die Ermittlung der Konzentration über der Zeit und der daraus folgenden Berechnung des Herzzeitvolumens ohnehin benötigte Rechner mit verwendet werden kann.

Die Erfindung betrifft ferner ein Verfahren zur Ermittlung des Herzzeitvolumens nach dem Oberbegriff des Anspruchs 8.

Diesbezüglich liegt ihr die Aufgabe zugrunde, ein Verfahren zur Ermittlung des Herzzeitvolumens dahingehend zu verbessern, daß die Belastung und Risiken für den Patienten wesentlich verringert werden und dennoch genaue Meßergebnisse erzielt werden können.

Diese Aufgabe wird bei einem Verfahren nach dem Oberbegriff des Anspruches 8 durch die im Kennzeichen angegebenen Merkmale gelöst.

Hinsichtlich der Wirkungsweise und Vorteile des Verfahrens gelten die Erläuterungen für die Vorrichtung entsprechend.

Weiterbildungen und vorteilhafte Ausgestaltung der Erfindung ergeben sich aus den weiteren Ansprüchen, der Beschreibung und der Zeichnungen, an Hand der die Erfindung erläutert wird.

In der Zeichnung zeigen:

Fig. 1 eine graphische Darstellung eines Konzentrationszeitverlaufs eines Indikatorfarbstoffes,

Fig. 2 ein Blockschaltbild einer erfindungsgemäßen Vorrichtung,

Fig. 3 eine graphische Darstellung des zeitlichen Verlaufes der Lichtintensitäten, wie sie mit einer Schaltung gemäß Fig. 2 ermittelt werden.

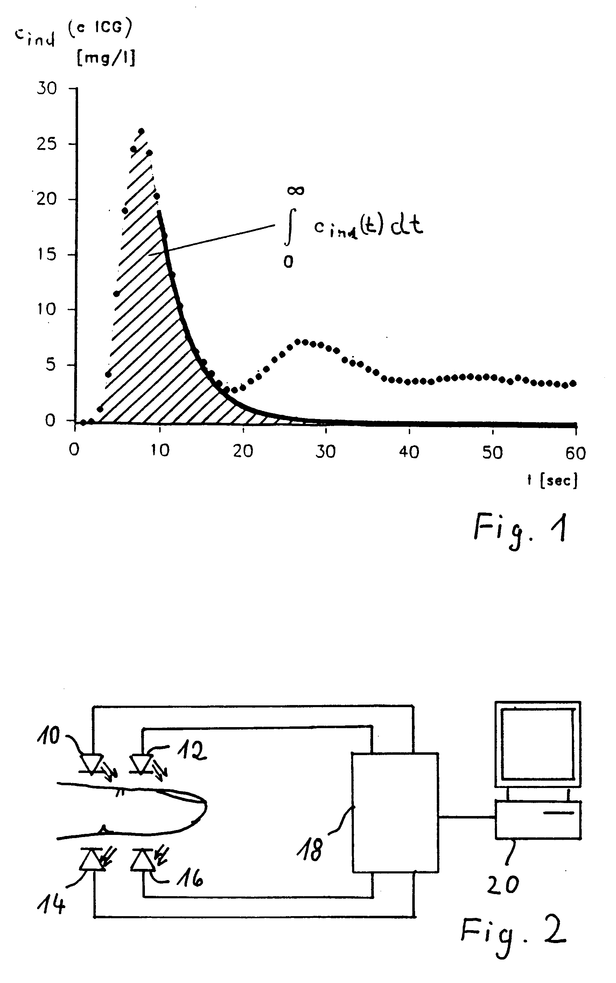

In Fig. 1 ist der Konzentrationszeitverlauf eines Indikatorfarbstoffs nach Injektion in den Blutkreislauf dargestellt. Es handelt sich um eine Indikatorverdünnungskurve bei einer Bolusinjektion von 25 mg Indocyaningrün in den rechten Vorhof des Herzens und aortaler Messung. Das nach dem ersten Maximum folgende zweite Maximum entsteht durch Rezirkulation, d.h. vor Abklingen der Konzentrationswelle bei einem ersten Durchlauf erfolgt bereits ein zweiter Durchlauf, der jedoch für die Messung außer Betracht bleiben muß. Zu diesem Zweck wird der abfallende Ast nach dem ersten Maximum, der nach Art einer e-Funktion weiterläuft, durch Extrapolation des mittleren Teils des abfallenden Astes ermittelt. Der gestrichelte Bereich unterhalb der gesamten ersten Konzentrationswelle entspricht dem Integral $c_{ind}(t)\, dt$, das in die Bestimmungsformel nach Stewart Hamilton

$$HZV = m_0\, /\, \int c_{ind}(t)\, dt$$

eingesetzt wird.

Eine ähnliche Verdünnungskurve zeigt auch der Konzentrationsverlauf bei Auswahl anderer Meß- und Injektionsorte. Dabei ist zu berücksichtigen, daß bei weit entfernt liegenden Meß- und Injektionsorten eine größere Verzögerungszeit vom Zeitpunkt der Injektion bis zu Auftreten der ersten Welle eintritt und auch die Amplitude kleiner ausfallen kann.

Um Einflüsse des verzögerten Indikatorfarbstoffeintritts in den jeweiligen Meßort kompensieren zu können, besteht die Möglichkeit, durchblutungsverbessernde Maßnahmen durchzuführen oder auch nachfolgende Wellen der Farbstoffkonzentrationszeitkurve unter Auswertung eines längeren Zeitabschnittes zu kompensieren. Dies kann durch erweiterte mathematische Auswertungsverfahren geschehen, wobei der gewählte Zeitabschnitt den Konzentrationsanstieg am Meßort und die Verteilungsphase des Farbstoffes im Blutkreislauf umfaßt. Man erhält dann durch Anwendung dieser Auswerteverfahren wieder einen von störenden Einflüssen bereinigten Kurvenverlauf der ersten Welle der Indikatorverdünnungskurve, dessen Integral zur Ermittlung des Herzzeitvolumens herangezogen werden kann.

Fig. 2 zeigt ein Blockschaltbild einer erfindungsgemäßen Vorrichtung. Dort sind zwei Lichtsender 10 und 12 und zwei Lichtempfänger 14 und 16 vorgesehen, die über eine Auswerteschaltung 18 mit einem Rechner 20 verbunden sind. Es sei davon ausgegangen, daß für die Messung des Herzzeitvolumens als Indikatorfarbstoff Indocyaningrün und als Referenzfarbstoff der rote Blutfarbstoff (Hämoglobin) verwendet wird. In diesem Fall arbeitet der eine Lichtsender 10 auf einer Wellenlänge von 800 Nanometern und der andere Lichtsender 12 auf einer Wellenlänge von 900 Nanometern. Die Lichtempfänger 14 und 16 können so ausgeführt sein, daß sie auch bevorzugt diese Wellenlängen herausfiltern. Alternativ kann aber auch zwischen zwei Lichtsendern 10 und 12 umgeschaltet werden und man kommt in diesem Fall mit nur einem Lichtempfänger 14 aus, der aber für beide Wellenlängen ausgelegt sein muß.

Die Messung des Absorptionsverhaltens der Farbstoffe kann sowohl durch Licht-Transmissions- oder auch durch Licht-Reflexionsmessung erfolgen. Geeignete Körperorte sind die Finger, die Ohrläppchen, der Nasenrücken, die Wangenschleimhaut oder die Stirn.

Die von den Lichtempfängern 14 und 16 empfangenen Signale der Lichtintensitäten werden von der Auswerteschaltung 18 ausgewertet und einem Rechner 20 zugeführt. In diesem Rechner 20 erfolgt zunächst eine Vorverarbeitung der pulsatilen Anteile der Lichtintensitäten $I_{ind\,puls}(t)$ und $I_{ref\,puls}(t)$, beispielsweise durch Quotientenbildung:

$$I_{ind\,puls}(t) = I_{ind}(t\text{-}dt) / I_{ind}(t\text{+}dt)$$

$$I_{ref\,puls}(t) = I_{ref}(t\text{-}dt) / I_{ref}(t\text{+}dt)$$

Aus diesen vorverarbeiteten Signalen kann in einem weiteren Schritt die sogenannte Meßwertgröße $M(t)$ gebildet werden, beispielsweise ebenfalls durch Quotientenbildung:

$$M(t) = I_{ind\,puls}(t) / I_{ref\,puls}(t)$$

Die Meßwertgröße weist einen kalibrierbaren stetigen Zusammenhang zum Verhältnis der beiden Farbstoffe in den pulsierenden Gefäßabschnitten $c_{ind/ref}(t)$, also in den Arterien und arteriellen Kapillaren, auf:

$$c_{ind/ref}(t) = f_{kal}(M(t))$$

Die Funktion $f_{kal}$ kann empirisch bestimmt werden und in Form einer Wertetabelle implementiert werden.

Zur Berechnung des Herzzeitvolumens muß die Indikator-Farbstoffkonzentration in absoluten Einheiten $c_{ind}(t)$ bekannt sein. Hierzu wird die Konzentration des Referenzfarbstoffes im Blut $c_{ref}$, z.B. des Hämoglobins, in einer Blutprobe bestimmt. Die Hämoglobinkonzentration im Blut gehört zu Routine-Laborparametern und ist bei den meisten Patienten bekannt oder kann relativ einfach bestimmt werden. Die gesuchte Konzentration des Indikatorfarbstoffes kann sodann mit Hilfe der bekannten Konzentration des Referenzfarbstoffes gemäß der Formel:

$$c_{ind}(t) = c_{ind/ref}(t) * c_{ref}$$

bestimmt werden. Der auf diese Weise ermittelte und ständig aktualisierte Wert $c_{ind}(t)$ für die Konzentration des Indikatorfarbstoffes wird in die Formel nach Stewart Hamilton

$$HZV = m_0 / \int c_{ind}(t)\, dt$$

eingesetzt, die schließlich den Wert für das Herzzeitvolumen HZV liefert.

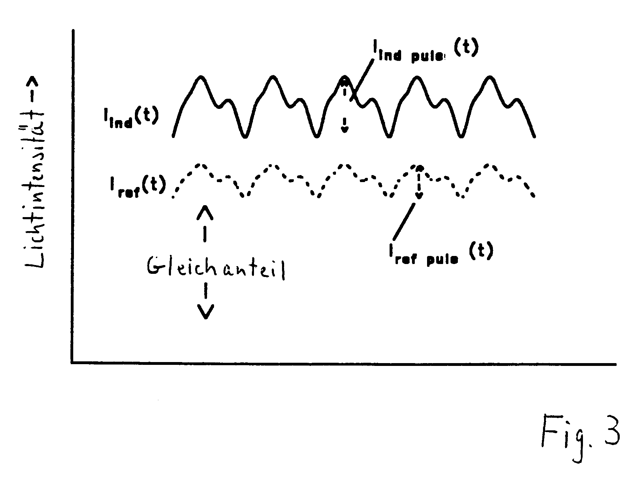

Fig. 3 zeigt den zeitlichen Verlauf der Lichtintensitäten, welcher mittels der Lichtempfänger 14 und 16 erfaßt und der Auswerteschaltung 18 zugeführt wird. Man erkennt hier einen Gleichanteil, der jeweils von einer periodischen Schwingung $I_{ind\,puls}(t)$ bzw. $I_{ref\,puls}(t)$ überlagert ist. Der Gleichanteil der Lichtintensität wird im wesentlichen durch die Absorptionseigenschaften des Gewebes und der nicht pulsierenden venösen Blutanteile determiniert. Die pulsatile Schwankungen in den Lichtintensitäten sind Folge der pulsatilen Blutströmung in arteriellen und kapillären Gefäßabschnitten.

Während eines Zeitraumes, in dem kein Indikatorfarbstoff im Blutkreislauf zirkuliert, sondern nur ein Referenzfarbstoff vorhanden ist, ist der pulsatile Anteil, der von beiden Lichtempfängern erfaßt wird, praktisch konstant. Wenn hingegen nach einer Injektion von Indikatorfarbstoff dieser zum Meßort gelangt, verändert sich vor allem die pulsatile Lichtintensität in dem Wellenlängenbereich, der im Absorptionsmaximum des Farbstoffes liegt.

Wird als Referenzfarbstoff der rote Blutfarbstoff benutzt, so läßt sich sein Anteil im Blut durch eine im Rahmen einer üblichen Blutuntersuchung durchzuführende Konzentrationsmessung in vitro ermitteln. Da dieser Wert eine recht hohe Langzeitkonstanz besitzt, kann davon ausgegangen werden, daß er auch während der gesamten Messung diesen zuvor ermittelten Wert beibehält und dieser somit eine verläßliche Grundlage für die Konzentrationsberechnung des Indikatorfarbstoffes bilden kann

Theoretisch wäre es auch möglich, einen anderen Referenzfarbstoff zu benutzen, der zuvor in den Blutkreislauf injiziert wurde und dessen Konzentration nach vollständiger Durchmischung ermittelt wurde. Auch wenn sich die Konzentration dieses Referenzfarbstoffes verändert, läßt sich seine Konzentration zur Ermittlung des Herzzeitvolumens heranziehen, da bei einer bekannten Konzentrationsänderung des Referenzfarbstoffes dieses Verhalten in die Auswertung einbezogen werden kann.

Statt der Messung des Lichtintensität bei zwei Wellenlängen kann eine Messung auch bei drei Wellenlängen erfolgen. Während die Messung mit dem Zwei-Wellenlängen-Verfahren für den Meßzeitraum eine Konstanz der arteriellen Sauerstoffsättigung voraussetzt, kann bei Verwendung von drei oder mehr Wellenlängen die Sauerstoffsättigung simultan ermittelt und bei der Auswertung berücksichtigt werden. Um mit dem Verfahren genaue Meßwerte ermitteln zu können, können Eichkurven für die ermittelten Farbstoffkonzentrationen bestimmt werden, die sich auf einen definierten Sensortyp und Meßort beziehen. Derartige Eichkurven lassen sich durch einen Vergleich mit photometrischen in vitro Bestimmungen der Farbstoffkonzentrationen ermitteln. Hierzu ist eine Injektion von Indikatorfarbstoff an Versuchspersonen vorgesehen, die einen intraindividuellen Vergleich der Methoden bei unterschiedlichen durch die Eliminationskinetik vorgegebenen Farbstoffkonzentrationen ermöglicht.

**Patentansprüche**

1. Vorrichtung zur Ermittlung des Herzzeitvolumens HZV, bestehend aus einer Meßvorrichtung (10, 12, 14, 16, 18), mit der nach Injizieren einer vorgegebenen Menge Indikatorfarbstoff $m_0$ in den Blutkreislauf eine Messung der Farbstoffkonzentration des Indikatorfarbstoffes durchgeführt wird, wobei die Meßvorrichtung Lichtsender (10, 12), Lichtempfänger (14, 16) und eine Auswerteschaltung (18) umfaßt, mit der nicht-invasiv mittels Licht-Transmissions- oder Reflexionsmessung eingestrahlten Lichtes an geeigneten Körperorten, insbesondere am Finger, Ohrläppchen, Nasenrücken, Wangenschleimhaut oder Stirn Lichtintensitäten bei mindestens zwei Wellenlängen ($I_{ind}(t)$, $I_{ref}(t)$) gemessen werden, und bestehend aus einem Rechner (20) zur Ermittlung des Herzzeitvolumens aus der vorgegebenen Menge des injizierten Indikatorfarbstoffes $m_0$ unter Bezug auf die bekannte Konzentration eines internen Referenzfarbstoffes $c_{ref}$, vorzugsweise des Hämoglobins, _dadurch gekennzeichnet,_ daß eine Messung der Farbstoffkonzentrationszeitkurve $c_{ind}(t)$ über einen ausreichenden Zeitraum bis wenigstens zum Eintritt einer ersten Rezirkulation erfolgt und das Herzzeitvolumen mittels des Rechners (20) aus der vorgegebenen Menge des injizierten Indikatorfarbstoffes $m_0$ und aus der Indikator-Farbstoffkonzentrationszeitkurve $c_{ind}(t)$, basierend auf dem Prinzip der Massenerhaltung gemäß der Formel nach Stewart Hamilton

$$HZV = m_0 / \int c_{ind}(t) \, dt$$

errechnet wird, wobei die Indikator-Farbstoffkonzentrationszeitkurve $c_{ind}(t)$ durch Messung der zeitlichen oder pulsatilen Schwankungen ($I_{ind\,puls}(t)$), $I_{ref\,puls}(t)$) der Lichtintensitäten bei den mindestens zwei Wellenlängen ($I_{ind}(t)$, $I_{ref}(t)$) und dem aus der Messung gewonnenen Verhältnis der beiden Farbstoffe in den pulsierenden Gefäßabschnitten $c_{ind/ref}(t)$ und der bekannten Konzentration des Referenzfarbstoffes $c_{ref}$ gemäß der Formel

$$c_{ind}(t) = c_{ind/ref}(t) \, {}^{*} \, c_{ref}$$

bestimmt wird.

2. Vorrichtung nach Anspruch 1, _dadurch gekennzeichnet,_ daß durch den Rechner (20) aus den zeitlichen oder pulsatilen Schwankungen der Lichtintensitäten $I_{ind\,puls}(t)$, $I_{ref\,puls}(t)$, insbesondere durch Bildung des Quotienten von $I_{ind\,puls}(t)$ und $I_{ref\,puls}(t)$, eine Meßwertgröße der Beziehung M(t) = $f_m$ ($I_{ind\,puls}(t)$, $I_{ref\,puls}(t)$) abgeleitet wird, wobei M(t) einen kalibrierbaren Zusammenhang $f_{kal}$ (M(t) in der Form einer monoton stetigen Funktion zum Verhältnis der intravaskulären Indikator-Farbstoffkonzentration zu einer Referenz-Farbstoffkonzentration $c_{ind/ref}(t)$ gemäß der Beziehung $c_{ind/ref}(t) = f_{kal}$ (M(t) aufweist und daß durch den Rechner (20) die absolute Konzentrationszeitkurve des Indikatorfarbstoffes $c_{ind}(t)$ über die bekannte intravaskuläre Konzentration des Referenzfarbstoffes $c_{ref}$, insbesondere durch Produktbildung mit der bekannten Referenz-Farbstoffkonzentration $c_{ref}$, nach der Beziehung $c_{ind}(t) = f_{c\,ref}(c_{ind/ref}(t), c_{ref})$ ermittelt wird.

3. Vorrichtung nach Anspruch 1 oder 2, _dadurch gekennzeichnet,_ daß die Meßvorrichtung (10, 12, 14, 16) selektiv auf wenigstens zwei Wellenlängen abgestimmt ist, bei denen der Indikatorfarbstoff und der Referenzfarbstoff unterschiedliches Absorptionsverhalten aufweisen.

4. Vorrichtung nach Anspruch 3, _dadurch gekennzeichnet,_ daß die auszuwertenden Wellenlängen des eingestrahlten Lichtes so gewählt sind, daß eine der Wellenlängen im Absorptionsbereich des injizierten Indikatorfarbstoffes liegt, und eine andere Wellenlänge in einem Bereich liegt, in dem der Referenzfarbstoff und der Indikatorfarbstoff unterschiedliches Absorptionsverhalten aufweisen.

**5.** Vorrichtung nach einem oder mehreren der Ansprüche 1 bis 4, dadurch gekennzeichnet, daß bei Auswertung von mehr als zwei Wellenlängen oder bei Auswertung eines kontinuierlichen Spektrums des eingestrahlten Lichtes die Konzentrationsverhältnisse mehrerer Farbstoffe in Relation zueinander oder in Relation zu einem Referenzfarbstoff ermittelt werden

**6.** Vorrichtung nach einem oder mehreren der Ansprüche 1 bis 4, dadurch gekennzeichnet, daß bei Verwendung von rotem Blutfarbstoff als Referenzfarbstoff und Indocyaningrün als Indikatorfarbstoff die Messung im maximalen Absorptionsbereich des Indocyaningrün (ca. 800 nm) und im nicht-absorbierenden infraroten Wellenlängenbereich des Indocyaningrüns (oberhalb von 900 nm) erfolgen.

**7.** Vorrichtung nach einem oder mehreren der Ansprüche 1 bis 6, dadurch gekennzeichnet, daß zur Kompensation der Einflüsse des verzögerten Indikatorfarbstoffeintritts in den jeweiligen Meßort, z.B. bei schlechter Durchblutung des jeweiligen Meßortes, vom Rechner (20) ein erweitertes mathematisches Auswertungsverfahren der Farbstoffkonzentrationszeitkurve des Indikatorfarbstoffes unter Auswertung eines längeren Zeitabschnittes durchgeführt wird, wobei dieser Zeitabschnitt so bemessen ist, daß er den Konzentrationsanstieg am Meßort und die Verteilungsphase des Farbstoffes, die Rezirkulation sowie beginnend die Elimination des Farbstoffes aus dem Blutkreislauf umfaßt.

**8.** Verfahren zur Ermittlung des Herzzeitvolumens HZV durch nicht-invasive Messung der Farbstoffkonzentration eines in einer vorgegebenen Menge in den Blutkreislauf injizierten Indikatorfarbstoffes $m_0$ mittels Licht-Transmissions- oder Reflexionsmessung eingestrahlten Lichtes an geeigneten Körperorten, insbesondere am Finger, Ohrläppchen, Nasenrücken, Wangenschleimhaut oder Stirn Lichtintensitäten bei mindestens zwei Wellenlängen ($I_{ind}(t)$, $I_{ref}(t)$) und Berechnung des Herzzeitvolumens aus der vorgegebenen Menge des injizierten Indikatorfarbstoffes $m_0$ unter Bezug auf die bekannte Konzentration eines internen Referenzfarbstoffes $c_{ref}$, vorzugsweise des Hämoglobins, dadurch gekennzeichnet, daß eine Messung der Indikator-Farbstoffkonzentrationszeitkurve $c_{ind}(t)$ über einen ausreichenden Zeitraum bis wenigstens zum Eintritt einer ersten Rezirkulation durchgeführt wird und das Herzzeitvolumen aus der vorgegebenen Menge des injizierten Indikatorfarbstoffes $m_0$ und aus der Indikator-Farbstoffkonzentrationszeitkurve $c_{ind}(t)$, basierend auf dem Prinzip der Massenerhaltung gemäß der Formel nach Stewart Hamilton

$$HZV = m_0 \, / \int c_{ind}(t) \, dt$$

errechnet wird, wobei die Indikator-Farbstoffkonzentrationszeitkurve $c_{ind}(t)$ durch Messung der zeitlichen oder pulsatilen Schwankungen ($I_{ind\;puls}(t)$, $I_{ref\;puls}(t)$) der Lichtintensitäten bei den mindestens zwei Wellenlängen ($I_{ind}(t)$, $I_{ref}(t)$) und dem aus der Messung gewonnenen Verhältnis der beiden Farbstoffe in den pulsierenden Gefäßabschnitten $c_{ind/ref}(t)$ und der bekannten Konzentration des Referenzfarbstoffes $c_{ref}$ gemäß der Formel

$$c_{ind}(t) = c_{ind/ref}(t) \; ^* \; c_{ref}$$

bestimmt wird.

**9.** Verfahren nach Anspruch 8, dadurch gekennzeichnet, daß aus den zeitlichen oder pulsatilen Schwankungen der Lichtintensitäten $I_{ind\;puls}(t)$, $I_{ref\;puls}(t)$, insbesondere durch Bildung des Quotienten von $I_{ind\;puls}(t)$ und $I_{ref\;puls}(t)$, eine Meßwertgröße der Beziehung $M(t) = f_m (I_{ind\;puls}(t), I_{ref\;puls}(t))$ abgeleitet wird, wobei $M(t)$ einen kalibrierbaren Zusammenhang $f_{kal}$ ($M(t)$ in der Form einer monoton stetigen Funktion zum Verhältnis der intravaskulären Indikator-Farbstoffkonzentration zu einer Referenz-Farbstoffkonzentration $c_{ind/ref}(t)$ gemäß der Beziehung $c_{ind/ref}(t) = f_{kal}$ ($M(t)$ aufweist und daß die absolute Konzentrationszeitkurve des Indikatorfarbstoffes $c_{ind}(t)$ über die bekannte intravaskuläre Konzentration des Referenzfarbstoffes $c_{ref}$, insbesondere durch Produktbildung mit der bekannten Referenz-Farbstoffkonzentration $c_{ref}$, nach der Beziehung $c_{ind}(t) = f_{c\;ref}(c_{ind/ref}(t), c_{ref})$ ermittelt wird.

**10.** Verfahren nach Anspruch 8 oder 9, dadurch gekennzeichnet, daß die Lichtintensitäten des eingestrahlten Lichtes von mindestens zwei Wellenlängen gemessen werden, bei denen die Farbstoffe unterschiedliches Absorptionsverhalten aufweisen, und die gemessenen Lichtintensitäten in Relation zueinander gesetzt werden.

**11.** Verfahren nach einem oder mehreren der Ansprüche 8 bis 10, dadurch gekennzeichnet, daß die auszuwertenden Wellenlängen des eingestrahlten Lichtes so gewählt werden, daß eine der Wellenlängen im Absorptionsbereich des injizierten Indikatorfarbstoffes liegt, und eine andere Wellenlänge in einem Bereich liegt, in dem der Referenzfarbstoff und der Indikatorfarbstoff unterschiedliches Absorptionsverhalten aufweisen.

**12.** Verfahren nach einem oder mehreren der Ansprüche 8 bis 11, dadurch gekennzeichnet, daß bei Auswertung von mehr als zwei Wellenlängen oder bei Auswertung eines kontinuierlichen Spektrums des eingestrahlten Lichtes die Konzentrationsverhältnisse mehrerer Farbstoffe in Relation zueinander oder in Relation zu einem Referenzfarbstoff ermittelt werden

13. Verfahren nach einem oder mehreren der Ansprüche 8 bis 12, dadurch gekennzeichnet, daß bei Verwendung von rotem Blutfarbstoff als Referenzfarbstoff und Indocyaningrün als Indikatorfarbstoff die Messungen im maximalen Absorptionsbereich des Indocyaningrün (ca. 800nm) und im nicht absorbierenden infraroten Wellenlängenbereich des Indocyaningrüns (oberhalb von 900 nm) durchgeführt werden.

14. Verfahren nach einem oder mehreren der Ansprüche 8 bis 13, dadurch gekennzeichnet, daß Einflüssen des verzögerten Indikatorfarbstoffeintritts in den jeweiligen Meßort, z.B. bei schlechter Durchblutung des jeweiligen Meßortes, durch durchblutungsverbessernde Maßnahmen entgegengewirkt wird.

15. Verfahren nach einem oder mehreren der Ansprüche 8 bis 13, dadurch gekennzeichnet, daß zur Kompensation der Einflüsse des verzögerten Indikatorfarbstoffeintritts in den jeweiligen Meßort, z.B. bei schlechter Durchblutung des jeweiligen Meßortes, ein erweitertes mathematisches Auswertungsverfahren der Farbstoffkonzentrationszeitkurve des Indikatorfarbstoffes unter Auswertung eines längeren Zeitabschnittes durchgeführt wird, wobei dieser Zeitabschnitt so bemessen ist, daß er den Konzentrationsanstieg am Meßort, die Verteilungsphase des Farbstoffes, die Rezirkulation sowie beginnend die Elimination des Farbstoffes aus Blutkreislauf umfaßt.

## Claims

1. Device for determining the heart minute volume HZV, consisting of a measuring device (10, 12, 14, 16, 18), by which, after the injection of a predetermined quantity of indicator dye $m_0$ into the blood stream, a measurement of the dye concentration of the indicator dye is performed, the measuring device comprising light emitter (10, 12), light receiver (14, 16) and an evaluating circuit (18), with which, by means of light transmission or reflection measurement of incident light at suitable locations of the body, especially at the finger, ear lobe, bridge of the nose, mucus membrane of the cheek or forehead, light intensities are non-invasively measured at at least two wavelengths ($I_{ind}(t)$, $I_{ref}(t)$), and consisting of a computer (20) for determining the heart minute volume from the predetermined quantity of the injected indicator dye $m_0$ with reference to the known concentration of an internal reference dye $c_{ref}$, preferably of the haemoglobin, characterized in that a measurement of the dye concentration time curve $c_{ind}(t)$ is carried out over a sufficient period until at least entry of a first recirculation, and the heart minute volume (HZV) is calculated by means of the computer (20) from the predetermined quantity of the injected indicator dye $m_0$ and from the indicator dye concentration time curve $c_{ind}(t)$, on the basis of the principle of the conservation of mass according to the formula of Stewart Hamilton

$$HZV = m_0 / \int c_{ind}(t)\, dt$$

wherein the indicator dye concentration time curve $c_{ind}(t)$ is determined by measurement of the fluctuations with time or with pulse ($I_{ind\ puls}(t)$), $I_{ref\ puls}(t)$) of the light intensities at the at least two wavelengths ($I_{ind}(t)$, $I_{ref}(t)$) and from the ratio obtained from the measurement of the two dyes in the pulsating vessel portions $c_{ind/ref}(t)$ and of the known concentration of the reference dye $c_{ref}$ according to the formula

$$c_{ind}(t) = c_{ind/ref}(t) * c_{ref.}$$

2. Device according to claim 1, characterized in that a measured value of the relationship $M(t) = f_m$ ($I_{ind\ puls}(t)$, $I_{ref\ puls}(t)$) is derived by the computer (20) from the fluctuations with time or with pulse of the light intensities $I_{ind\ puls}(t)$, $I_{ref\ puls}(t)$, especially by forming the quotient of $I_{ind\ puls}(t)$ and $I_{ref\ puls}(t)$, where $M(t)$ possesses a calibrated relationship $f_{kal}$ ($M(t)$ in the form of a monotonously continuous function to the ratio of the intravascular indicator dye concentration to a reference dye concentration $c_{ind/ref}(t)$ according to the relationship $c_{ind/ref}(t) = f_{kal} M(t)$ and that the absolute concentration time curve of the indicator dye $c_{ind}(t)$ is calculated by the computer (20) from the known intravascular concentration of the reference dye $c_{ref}$, especially by forming the product with the known reference dye concentration $c_{ref}$, according to the relationship $c_{ind}(t) = f_{c\ ref}$ ($C_{ind/ref}(t)$, $c_{ref}$).

3. Device according to claim 1 or 2, characterized in that the measuring device (10, 12, 14, 16) is selectively tuned to at least two wavelengths, at which the indicator dye and the reference dye exhibit different absorption behaviours.

4. Device according to claim 3, characterized in that the wavelengths of the incident light to be evaluated are so chosen that one of the wavelengths lies in the absorption range of the injected indicator dye and another wavelength lies in a range, in which the reference dye and the indicator dye exhibit different absorption behaviours.

5. Device according to one or more of claims 1 to 4, characterized in that, in the evaluation of more than two wavelengths or in the evaluation of a continuous spectrum of the incident light, the concentration ratios of several dyes in relation to one another or in relation to a reference dye are determined.

6. Device according to one or more of claims 1 to 4, characterized in that, where red haemoglobin is used as reference dye and indocyanine green as indicator dye, the measurement takes place in the maximum absorption range of indocyanine green (approx. 800 nm) and in the non-absorbing infra red wavelength range of indocyanine green (above 900 nm).

7. Device according to one or more of claims 1 to 6, characterized in that, for compensating the influences of the delayed indicator dye entry into the particular measurement location, for example in the case of bad circulation through the particular measurement location, an extended mathematical evaluation method of the dye concentration time curve of the indicator dye is carried out by the computer (20) with evaluation of a longer time portion, wherein this time portion is so dimensioned that it contains the concentration rise at the measurement location and the distribution phase of the dye, the recirculation and also commencement of the elimination of the dye from the blood stream.

8. Method of determining the heart minute volume HZV by non-invasive measurement of the dye concentration of an indicator dye $m_0$ injected in a predetermined quantity into the blood stream by means of light transmission or reflection measurement of incident light at suitable body locations, especially at the finger, lobe of the ear, bridge of the nose, mucus membrane of the cheek or the forehead, of light intensities at at least two wavelengths ($I_{ind}(t)$, $I_{ref}(t)$) and calculation of the heart minute volume from the predetermined quantity of the injected indicator dye $m_0$ with reference to the known concentration of an internal reference dye $c_{ref}$, preferably of the haemoglobin, characterized in that a measurement of the indicator dye concentration time curve $c_{ind}(t)$ is carried out over a sufficient period until at least entry of a first recirculation and the heart minute volume is determined from the predetermined quantity of the injected indicator dye $m_0$ and from the indicator dye concentration time curve $c_{ind}(t)$, on the basis of the principle of the conservation of mass according to the formula of Stewart Hamilton

$$HZV = m_0 \, / \int c_{ind}(t) \, dt,$$

the indicator dye concentration time curve $c_{ind}(t)$ being determined by measurement of the fluctuations with time or with pulses ($I_{ind\,puls}(t)$, $I_{ref\,puls}(t)$) of the light intensities at the at least two wavelengths ($I_{ind}(t)$, $I_{ref}(t)$) and the ratio, obtained from the measurement, of the two dyes in the pulsating vessel portions $c_{ind/ref}(t)$ and of the known concentration of the reference dye $c_{ref}$ according to the formula

$$c_{ind}(t) = c_{ind/ref}(t) \, ^* \, c_{ref}.$$

9. Method according to claim 8, characterized in that a measured value of the relationship $M(t) = f_m \, (I_{ind\,puls}(t), \, I_{ref\,puls}\,(t))$ is derived from the fluctuations, with time or pulses, of the light intensities $I_{ind\,puls}(t)$, $I_{ref\,puls}(t)$, especially by forming the quotient of $I_{ind\,puls}(t)$ and $I_{ref\,puls}(t)$, where $M(t)$ has a calibrated relationship $f_{kal}\,M(t)$ in the form of a monotonously continuous function to the ratio of the intravascular indicator dye concentration to a reference dye concentration $c_{ind/ref}(t)$ according to the relationship $c_{ind/ref}(t) = f_{kal}\,M(t)$, and that the absolute concentration time curve of the indicator dye $c_{ind}(t)$ is determined from the known intravascular concentration of the reference dye $c_{ref}$, especially by product formation with the known reference dye concentration $c_{ref}$, according to the relationship $c_{ind}(t) = f_{c\,ref}(c_{ind/ref}(t), \, c_{ref})$.

10. Method according to claim 8 or 9, characterized in that the light intensities of the incident light of at least two wavelenghts are measured, at which the dyes exhibit different absorption behaviours, and the measured light intensities are set in relationship to one another.

11. Method according to one or more of claims 8 to 10, characterized in that the wavelengths of the incident light to be evaluated are so chosen that one of the wavelengths lies in the absorption range of the injected indicator dye, and another wavelength lies in a range, in which the reference dye and the indicator dye exhibit different absorption behaviours.

12. Method according to one or more of claims 8 to 11, characterized in that, in the evaluation of more than two wavelengths or in the evaluation of a continuous spectrum of the incident light, the concentration ratios of several dyes in relation to one another or in relation to a reference dye are determined.

13. Method according to one or more of claims 8 to 12, characterized in that, where red haemoglobin is used as reference dye and indocyanine green as indicator dye, the measurements are carried out in the maximum absorption range of indocyanine green (approx. 800 nm) and in the non-absorbing infra red wavelength range of indocyanine green (above 900 nm).

14. Method according to one or more of claims 8 to 13, characterized in that influences of the delayed indicator dye entry into the particular measurement location, for example in the case of bad circulation through the relevant measurement location, are counteracted by circulation improving measures.

15. Method according to one or more of claims 8 to 13, characterized in that, for compensating the influences of the delayed indicator dye entry into the particular measurement location, for example in the case of bad circulation of the relevant measurement location, an extended mathematical evaluation method of the dye concentration time curve of the indicator dye is carried out with evaluation of a longer time portion, this time portion being so dimensioned that it contains the concentration increase at the measurement location, the distribution phase of the dye, the recirculation and the commencement of the elimination of the dye from the blood circuit.

## Revendications

1. Dispositif pour déterminer le volume de temps du coeur HZV, composé d'un dispositif de mesure (10, 12, 14, 16, 18), avec lequel, après injection d'une quantité préterminée d'un produit colorant indicateur $m_0$ dans le circuit sanguin, est réalisée une mesure de la concentration de produit colorant indicateur, le dispositif de mesure comprenant des émetteurs de lumière (10, 12), des récepteurs de lumière (14, 16) et un circuit d'évaluation (18), avec lequel, de façon non invasive, au moyen de mesure de transmission ou de réflexion de lumière irradiée en des points du corps appropriés, en particulier sur le doigt, le lobe de l'oreille, le dos du nez, les muqueuses de joue ou le front, des intensités de lumière sont mesurées pour au moins deux longueurs d'onde ($I_{ind}(t)$, $I_{réf}(t)$), et d'un calculateur (20) pour déterminer le volume de temps du coeur à partir de la quantité prédéterminée de produit colorant indicateur injecté $m_0$ par rapport à la concentration connue d'un produit colorant de référence interne $C_{réf}$, de préférence de l'hémoglobine, caractérisé en ce qu'une mesure de la courbe temporelle de la concentration de couleur $c_{ind}(t)$ sur un espace de temps suffisant jusqu'à au moins l'entrée d'une première recirculation suit et que le volume éjecté par minute de coeur est calculé à partir de la quantité prédéterminée de produit colorant indicateur injecté $m_0$ et de la courbe temporelle de concentration en colorant indicateur $c_{ind}(t)$, en se basant sur le principe de conservation de là masse suivant la formule de Stewart Hamilton

$$HVZ = m_0 / \int c_{ind}(t)\, dt$$

la courbe temporelle de concentration en colorant indicateur $c_{ind}(t)$ étant estimée par mesure de fluctuations temporelles ou pulsées ($I_{ind\,puls}(t)$, $I_{réf\,puls}(t)$) des intensités de lumière pour au moins les deux longueurs ($I_{ind}(t)$, $I_{réf}(t)$) et la proportion obtenue à partir de la mesure des deux produits colorants en segments de réservoir pulsants $c_{ind/réf}(t)$ et de la concentration connue du produit colorant de référence $c_{réf}$ suivant la formule

$$c_{ind}(t) = c_{ind/réf}(t) * c_{réf}$$

2. Dispositif suivant la revendication 1, caractérisé en ce que, avec le calculateur (20) à partir des fluctuations temporelles ou pulsées des intensités de lumière $I_{ind\,puls}(t)$, $I_{réf\,puls}(t)$, en particulier par création du quotient de $I_{ind\,puls}(t)$ et $I_{réf\,puls}(t)$, est dérivée une valeur de mesure du rapport $M(t) = f_m (I_{ind\,puls}(t), I_{réf\,puls}(t))$, $M(t)$ représentant une relation pouvant être calibrée $f_{cal}\, M(t)$ sous la forme d'une fonction continue monotone en proportion de la concentration de couleur indicateur intravasculaire à une concentration de couleur de référence $c_{ind/réf}(t)$, suivant la relation $c_{ind/réf}(t) = f_{cal}\, M(t)$ et en ce que, avec le calculateur (20), est déterminée la courbe temporelle de la concentration absolue du produit colorant indicateur $c_{ind}(t)$ par la concentration intravasculaire connue du produit colorant de référence $c_{réf}$, en particulier par formation de produit comportant la concentration de couleur de référence connue $c_{réf}$, d'après la relation $c_{ind}(t) = f_{créf}(c_{ind/réf}(t), c_{réf})$.

3. Dispositif suivant la revendication 1 ou 2, caractérisé en ce que le dispositif de mesure (10, 12, 14, 16) est adapté, de manière sélective, sur au moins deux longueurs d'onde pour lesquelles le produit colorant indicateur et le produit colorant de référence présentent des caractéristiques d'absorption différentes.

4. Dispositif suivant la revendication 3, caractérisé en ce que les longueurs d'onde à évaluer de la lumière irradiée sont choisies de façon qu'une des longueurs d'onde soit dans le domaine d'absorption du produit colorant indicateur et que l'autre soit dans un domaine dans lequel le produit colorant de référence et le produit colorant indicateur présentent des caractéristiques d'absorption différentes.

5. Dispositif suivant une ou plusieurs des revendications 1 à 4, caractérisé en ce que, par évaluation de plus de deux longueurs d'onde ou par évaluation d'un spectre continu de la lumière irradiée, les proportions de concentration de plusieurs produits colorants sont déterminées en relation l'une avec l'autre, ou en relation avec un produit colorant de référence.

6. Dispositif suivant une ou plusieurs des revendications 1 à 4, caractérisé en ce qu'en utilisant un produit de couleur rouge sang comme produit colorant de référence et de l'indocyanine vert comme produit colorant indicateur, les mesures sont réalisées dans le domaine d'absorption maximal d'indocyanine vert (à environ 800 nm) et dans le domaine des longueurs d'onde infrarouge non-absorbantes d'indocyanine vert (au-dessus de 900 nm).

7. Dispositif suivant une ou plusieurs des revendications 1 à 6, <u>caractérisé en ce</u> que, pour la compensation de l'influence de l'entrée de produit colorant indicateur retardée au point correspondant de mesure, par exemple par mauvaise pénétration au point correspondant de mesure, est réalisé avec le calculateur (20) un procédé d'évaluation mathématique extensif de la courbe temporelle de la concentration du produit colorant indicateur en exploitant un intervalle de temps plus long, cet intervalle étant mesuré de façon qu'il comprenne l'augmentation de concentration au point de mesure et la phase de partage du produit colorant, la recirculation ainsi que le début de l'élimination du produit hors du circuit de circulation sanguine.

8. Procédé pour déterminer le volume de temps du coeur HZV par mesure non invasive de la concentration de produit colorant d'un produit colorant indicateur injecté en quantité prétérminée $m_0$ dans le circuit sanguin, au moyen de mesure de transmission ou de réflexion de lumière irradiée en des points du corps appropriés, en particulier sur le doigt, le lobe de l'oreille, le dos du nez, les muqueuses de joue ou le front, pour au moins deux longueurs d'onde ($I_{ind}(t)$, $I_{réf}(t)$), et calcul du volume de temps du coeur à partir de la quantité prédéterminée de produit colorant indicateur injecté $m_0$ par rapport à la concentration connue d'un produit colorant de référence interne $C_{réf}$, de préférence de l'hémoglobine, <u>caractérisé en ce</u> qu'est réalisée une mesure de la courbe temporelle de la concentration de couleur $c_{ind}(t)$ sur un espace de temps suffisant jusqu'à au moins l'entrée d'une première recirculation et qu'est calculé le volume de temps du coeur à partir de la quantité prédéterminée de produit colorant indicateur injecté $m_0$ et de la courbe temporelle de concentration en colorant indicateur $c_{ind}(t)$, en se basant sur le principe de conservation de la masse suivant la formule de Stewart Hamilton

$$HVZ = m_0 \, / \int c_{ind}(t) \, dt$$

la courbe temporelle de concentration en colorant indicateur $c_{ind}(t)$ étant estimée par mesure de fluctuations temporelles ou pulsées ($I_{ind\ puls}(t)$, $I_{réf\ puls}(t)$) des intensités de lumière pour au moins les deux longueurs ($I_{ind}(t)$, $I_{réf}(t)$) et la proportion obtenue à partir de la mesure des deux produits colorants en segments de réservoir pulsants $c_{ind/réf}(t)$ et de la concentration connue du produit colorant de référence $c_{réf}$ suivant la formule

$$c_{ind}(t) = c_{ind/réf}(t) \, {}^* \, c_{réf}$$

9. Procédé suivant la revendication 8, <u>caractérisé en ce</u> que, à partir des fluctuations temporelles ou pulsées des intensités de lumière $I_{ind\ puls}(t)$, $I_{réf\ puls}(t)$, en particulier par création du quotient de $I_{ind\ puls}(t)$ et $I_{réf\ puls}(t)$, est dérivée une valeur de mesure du rapport $M(t) = f_m(I_{ind\ puls}(t), I_{réf\ puls}(t))$, $M(t)$ représentant une relation pouvant être calibrée $f_{cal}\ M(t)$ sous la forme d'une fonction continue monotone en proportion de la concentration de couleur indicateur intravasculaire à une concentration de couleur de référence $c_{ind/réf}(t)$, suivant la relation $c_{ind/réf}(t) = f_{cal}\ M(t)$ et en ce qu'est déterminée la courbe temporelle de la concentration absolue du produit colorant indicateur $c_{ind}(t)$ par la concentration intravasculaire connue du produit colorant de référence $c_{réf}$, en particulier par formation de produit comportant la concentration de couleur de référence connue $c_{réf}$, d'après la relation $c_{ind}(t) = f_{créf}(c_{ind/réf}(t), c_{réf})$.

10. Procédé suivant la revendication 8 ou 9, <u>caractérisé en ce</u> que les intensités de la lumière irradiée sont mesurées sur au moins deux longueurs d'onde pour lesquelles les produits colorants indicateurs présentent des caractéristiques d'absorption différentes et les intensités de lumière mesurées sont placées en relation l'une avec l'autre.

11. Procédé suivant une ou plusieurs des revendications 8 à 10, <u>caractérisé en ce</u> que les longueurs d'onde à évaluer de la lumière irradiée sont choisies de façon qu'une des longueurs d'onde soit dans le domaine d'absorption du produit colorant indicateur et que l'autre soit dans un domaine dans lequel le produit colorant de référence et le produit colorant indicateur présentent des caractéristiques d'absorption différentes.

12. Procédé suivant une ou plusieurs des revendications 8 à 11, <u>caractérisé en ce</u> que, par évaluation de plus de deux longueurs d'onde ou par évaluation d'un spectre continu de la lumière irradiée, les proportions de concentration de plusieurs produits colorants sont déterminées en relation l'une avec l'autre, ou en relation avec un produit colorant de référence.

13. Procédé suivant une ou plusieurs des revendications 8 à 12, <u>caractérisé en ce</u> qu'en utilisant un produit de couleur rouge sang comme produit colorant de référence et de l'indocyanine vert comme produit colorant indicateur, les mesures sont réalisées dans le domaine d'absorption maximal d'indocyanine vert (à environ 800 nm) et dans le domaine des longueurs d'onde infrarouge non-absorbantes d'indocyanine vert (au-dessus de 900 nm).

**14.** Procédé suivant une ou plusieurs des revendications 8 à 13, caractérisé en ce que l'influence de l'entrée de produit colorant indicateur retardée au point de mesure correspondant, par exemple par mauvaise pénétration au point correspondant de mesure, est contrariée par des mesures d'amélioration de pénétration.

**15.** Procédé suivant une ou plusieurs des revendications 8 à 13, caractérisé en ce que, pour la compensation de l'influence de l'entrée de produit colorant indicateur retardée au point correspondant de mesure, par exemple par mauvaise pénétration au point correspondant de mesure, est réalisé un procédé d'évaluation mathématique extensif de la courbe temporelle de la concentration du produit colorant indicateur en exploitant un intervalle de temps plus long, cet intervalle étant mesuré de façon qu'il comprenne l'augmentation de concentration au point de mesure et la phase de partage du produit colorant, la recirculation ainsi que le début de l'élimination du produit hors du circuit de circulation sanguine.

$$\int_{0}^{\infty} c_{ind}(t)\, dt$$

Fig. 1

Fig. 2

Fig. 3